# EUROPEAN PATENT APPLICATION

(11) **EP 1 785 432 A1**
(43) Date of publication of application: **16.05.2007**
(21) Application number: 05024961.4
(22) Date of filing: 15.11.2005
(51) Int. Cl.: C07K 7/56, C07K 1/113

(54) **Process and intermediates for the synthesis of caspofungin.**

(71) Applicant: SANDOZ AG, 4056 Basel (CH)
(72) Inventor: Ludescher, Johannes, 6252 Breitenbach (AT); Macher, Ingolf, 6300 Wörgl (AT); Storm, Ole, 6330 Kufstein (AT); Bertel, Stephan, 6250 Kundl (AT)
(74) Representative: Dietz, Jörg-Reimar

(57) **Abstract**

The present invention relates to processes for preparing certain aza cyclohexapeptide compounds, e.g. caspofungin, novel intermediates used in said processes and a process for preparing said intermediates.
In particular, the intermediates have the following formula, wherein X is amino, substituted amino or ether, and contain a cyano (nitrile) functionality.

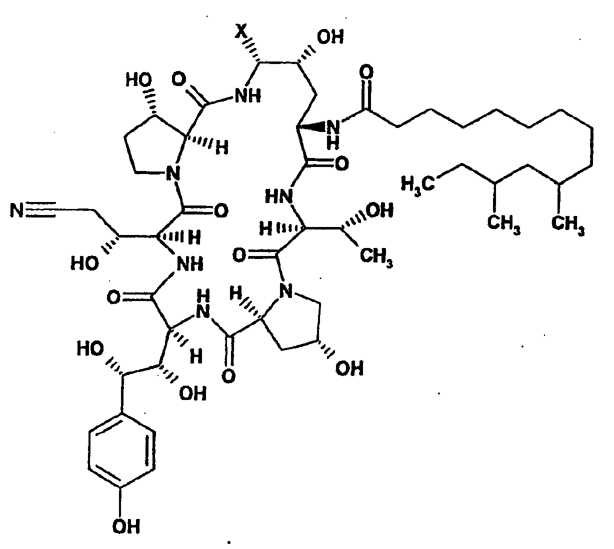

## Description

The present invention relates to processes for preparing certain aza cyclohexapeptide compounds, novel intermediates used in said processes and a process for preparing said intermediates.

Aza cyclohexapeptide compounds of formula I as defined below are macrocyclic lipopeptides belonging to the echinocandin family which are useful in treating systemic fungal infections, especially those caused by *Candida, Aspergillus, Histoplasma, Coccidioides* and *Blastomyces.* They have also been found useful for the treatment and prevention of infections caused by *Pneumocystis carinii* which are often found in immunocompromised patients such as those with AIDS. Pneumocandins are a subset of echinocandins which are naturally produced by the fungus *Glarea lozoyensis.* Their isolation, structure elucidation and biological evaluation have been reported by Schmatz et al in Cutaneous Antifungal Agents, 1993, pp375-394. Pneumocandin Bo is a secondary metabolite produced the fungus *Glarea lozoyensis* and serves as an intermediate in the production of Caspofungin (see e.g. US patents no. 5194377 and 5202309).

The compound 1-[(4R,5S)-5-[(2-Aminoethyl)amino]-N2-(10,12-dimethyl-1-oxotetradecyl)-4-hydroxy-L-ornithine]-5-[(3R)-3-hydroxy-L-ornithine]-pneumocandin Bo and its pharmaceutical acceptable salts are known under the INN caspofungin to be useful in treating fungal infections (see Merck Index, 13th edition, monograph no. 1899).

Processes for the preparation of aza cyclohexapeptide compounds and caspofungin are described in e.g. WO94/21677, EP620232, WO96/24613, US5552521, WO97/47645, US5936062, WO02/083713. However, known processess are not optimal for industrial production in terms of yield, purity, stability and amount of side-products. There is the necessity to operate under strictly anhydrous conditions, e.g. by using molecular sieves. In addition the use of protecting groups is required to achieve the desired purity. Several chromatographic steps are necessary to purify intermediates as well as the final product. Therefore, there is a need for an improved process to prepare aza cyclohexapeptide compounds in an economic way applicable in industrial scale.

The present invention provides a process for preparing aza cyclohexapeptide compounds or pharmaceutically acceptable salts thereof in high yield and high purity. The process of the present invention is easily applicable and is able to be scaled up easily, e.g. to an industrial scale.

Accordingly, in one aspect the present invention relates to a process for the production of aza cyclohexapeptide compounds of formula I 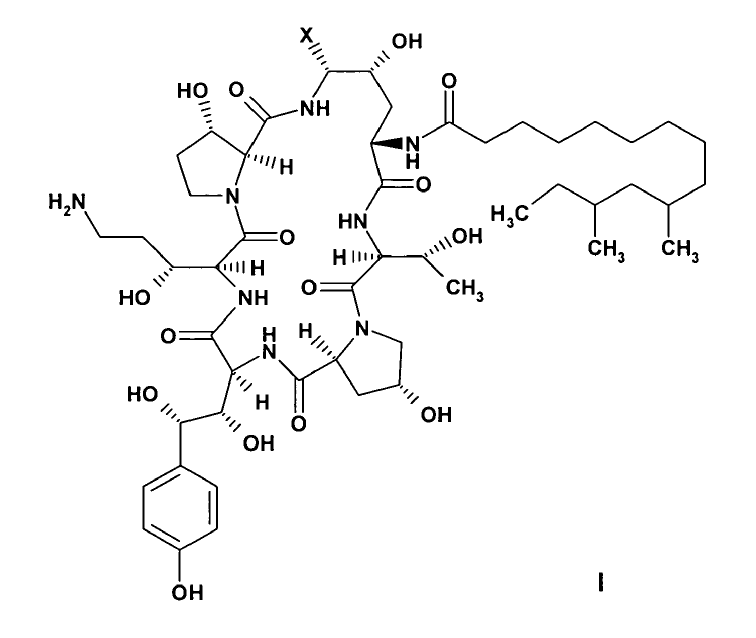 wherein
X is NR₁R₂ or OR₁;
and wherein when X is NR₁R₂
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
and wherein when X is OR₁
R₂, R₃ and R₄ are as defined above with the proviso that R₁ is not H;
or a pharmaceutically acceptable salt thereof comprising the steps of
a) reducing a compound of formula II or acid addition salts thereof 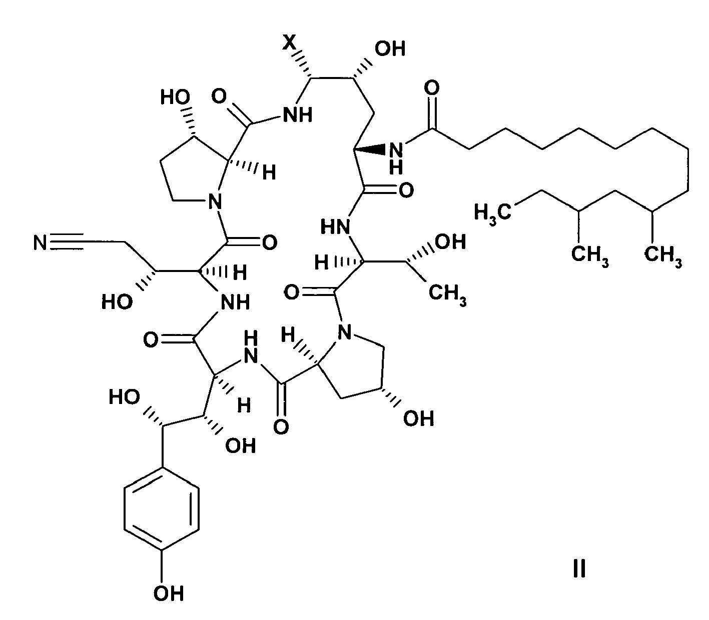 wherein X is defined as above,
   to obtain a compound of formula I or a pharmaceutically acceptable salt thereof, and
b) optionally isolating the compound of formula I or a pharmaceutically acceptable salt thereof as obtained in step a).

In preferred embodiments of the above process
R₁ is H and R₂ is selected from H, C₁-C₈ alkyl, C₃-C₄alkenyl, (CH₂)₂₋₄OH, (CH₂)₂₋₄NR₃R₄; or
R₁ is C₁-C₈ alkyl and R₂ is selected from H, C₁-C₈ alkyl,C₃-C₄alkenyl, (CH₂)₂₋₄OH, (CH₂)₂₋₄NR₃R₄; or
R₁ is C₃-C₄ alkenyl and R₂ is selected from H, C₁-C₈ alkyl,C₃-C₄ alkenyl, (CH₂)₂₋₄OH, (CH₂)₂₋₄NR₃R₄; or
R₁ is (CH₂)₂₋₄OH and R₂ is selected from H, C₁-C₈ alkyl, C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₈ alkyl,C₃-C₄ alkenyl, (CH₂)₂₋₄OH, (CH₂)₂₋₄NR₃R₄.

In a further preferred embodiment of the above process X is HN-CH₂-CH₂-NH₂.

In a further preferred embodiment of the above process aza cyclohexapeptide compounds of formula I are produced wherein
X is NR₁R₂ or OR₁;
and wherein when X is NR₁R₂
R₁ is H, C₁-C₄ alkyl , C₃-C₄ alkenyl, (CH₂)₂₋₄OH or (CH₂)₂₋₄NR₃R₄;
R₂ is H, C₁-C₄ alkyl, C₃-C₄alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₄ alkyl;
R₄ is H or C₁-C₄ alkyl;
and wherein when X is OR₁
R₂, R₃ and R₄ are as defined above with the proviso that R₁ is not H;

In preferred embodiments of the above process
R₁ is H and R₂ is selected from H, C₁-C₄ alkyl, C₃-C₄ alkenyl, (CH₂)₂₋₄OH, (CH₂)₂₋₄NR₃R₄; or
R₁ is C₁-C₄ alkyl and R₂ is selected from H, C₁-C₄ alkyl,C₃-C₄ alkenyl, (CH₂)₂₋₄OH, (CH₂)₂₋₄NR₃R₄; or
R₁ is C₃-C₄ alkenyl and R₂ is selected from H, C₁-C₄ alkyl,C₃-C₄ alkenyl, (CH₂)₂₋₄OH, (CH₂)₂₋₄NR₃R₄; or
R₁ is (CH₂)₂₋₄OH and R₂ is selected from H, C₁-C₄ alkyl,C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₄ alkyl,C₃-C₄ alkenyl, (CH₂)₂₋₄OH, (CH₂)₂₋₄NR₃R₄.

Optionally, X may be protected in order to archieve the desired regioselectivity , e.g if X contains several reactive nuclephilic groups, by protective groups such as amine or hydroxyl protecting groups. Said protective groups may be removed before, simultaneously or after the reduction step.

The reduction of compounds of formula II or acid addition salts thereof in step a) to obtain compounds of formula I may be performed by using any nitrile reducing agens. Preferably, catalytic hydrogenation is applied. Catalysts suitable for the reduction are e.g. catalysts originating from noble metals e.g. palladium, platinum, rhodium, ruthenium, e.g. HRh(PR₃)₃ wherein R represents an optionally substituted phenylgroup or an alkylgroup, e.g. isopropyl, Rh/Al₂O₃, ClR(PR₃)₃, Pt, PtO₂, Pd, Pd on carbon or Ru or RuCl₂ or catalyst such as nickel. Preferably, Rh/Al₂O₃ or Pd on carbon is used as catalyst. The hydrogen source may be H₂ or generated in situ e.g. from ammonium formiate or diimine reactions. Preferably, H₂ is used.

The amount of catalyst used in the reduction step may vary from 5% to 500% weight based on the compound of formula II.

Applied pressures may vary from atmospheric pressure up to 20 bar , e.g. from atmospheric pressure up to 10 bar.

The reduction step may be performed by dissolving or suspending compounds of formula II or acid addition salts thereof in a suitable solvent. Suitable solvents are solvents which are inert to reduction. Such solvents may be identified by a skilled person in routine tests. Suitable solvents are e.g. alcohols such as C₁-C₄ alcohols, e.g. methanol, ethanol or isopropanole, amides such as N,N-Dimethylformamide or n-methylpyrrolidon, optionally in combination with water. A preferred solvent is a mixture of isopropanol and water. Preferably, the amount of water is more than 5% of the amount of isopropanol. Optionally an acid e.g. acetic acid may be present in the reduction step to suppress impurity formation.

After the reduction step is complete the product may be isolated in step b) by methods known in the art. For instance, the catalyst may be filtered off and the product may be purified by chromatography, e.g reversed phase chromatography (e.g. a RP-8 or RP-18 type modified silica gel). Isolation from the fractions may be done by lyophilisation and the products may be isolated as free amines or as their addition salts with organic acids such as e.g. formic , acetic or trifluoroacetic acid. Caspofungin is preferably isolated as its diacetate.

Surprisingly, the reduction of the nitrile is of high selectivity in the presence of the other functionalities, e.g. in the presence of the aminal moiety or substituted hemiaminal present in the aza cyclohexapeptide compounds.

Therefore, in a further embodiment the present invention relates to a process of reducing a nitrile group to an amino group in a compound further containing an aminal moiety or a substituted hemiaminal moiety, in particular an oxygen substituted hemiaminal moiety, by catalytic hydrogenation. In particluar, said compound is an aza cyclohexapeptide compound of formula I or a pharmaceutically acceptable salt thereof. Preferably, said compound is caspofungin.
Thus, the invention also relates to the use of catalytic hydrogenation for the conversion of a nitrile group to an amino group in a compound further containing an aminal moiety or a substituted hemiaminal moiety, in particular an oxygen substituted hemiaminal moiety. The compounds mentioned above are preferred compounds for this use.

In addition the isolation and crystallization of the compound of formula VI allows an easy removal of the epimeric compound at C₃₅ amongst the removal of other impurities.

As starting material for said process Pneumocandin Bo may be used. Pneumocandin Bo may be further processed by dehydration, reaction with a thiophenol and substitution of the thiophenol group to obtain a compound of formula II.

Therefore, the present invention also relates to a process for the production of aza cyclohexapeptide compounds of formula I 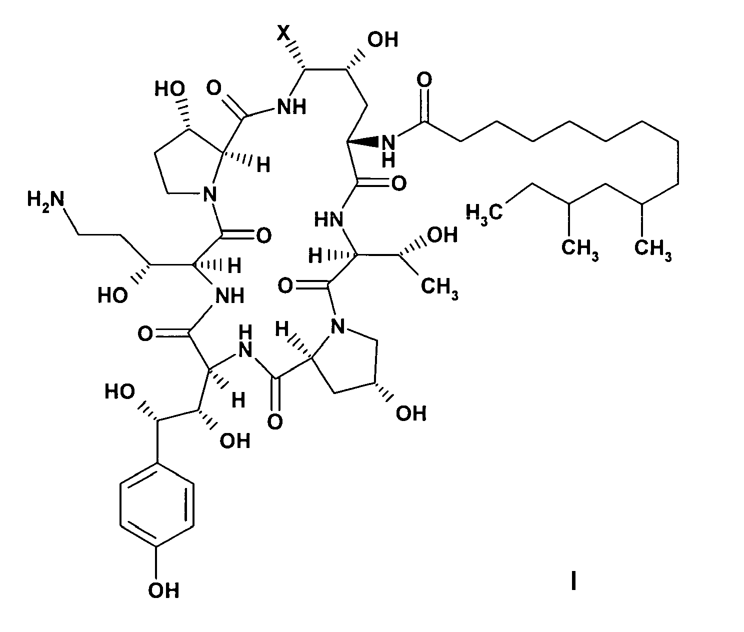 wherein
X is NR₁R₂ or OR₁;
and wherein when X is NR₁R₂
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
and wherein when X is OR₁
R₂, R₃ and R₄ are as defined above with the proviso that R₁ is not H;
or a pharmaceutically acceptable salt thereof comprising the steps of
a) reacting a compound of formula III 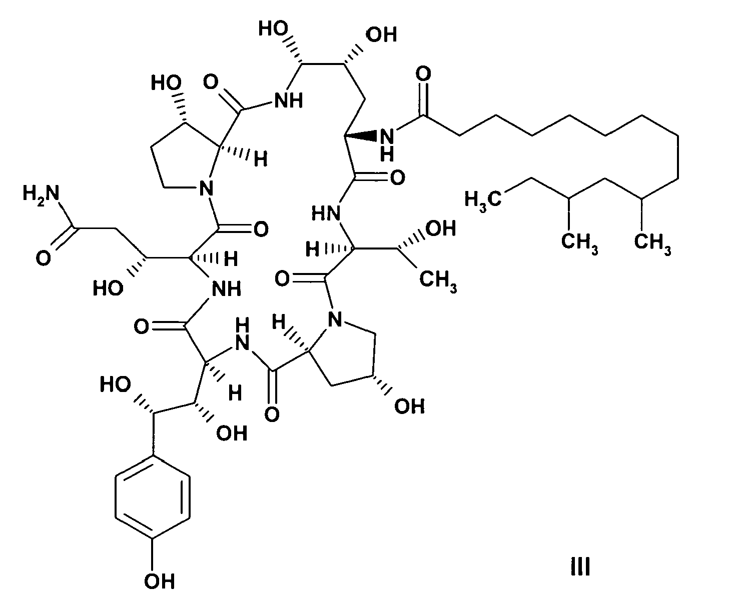 with a dehydrating agent to obtain a compound of formula IV 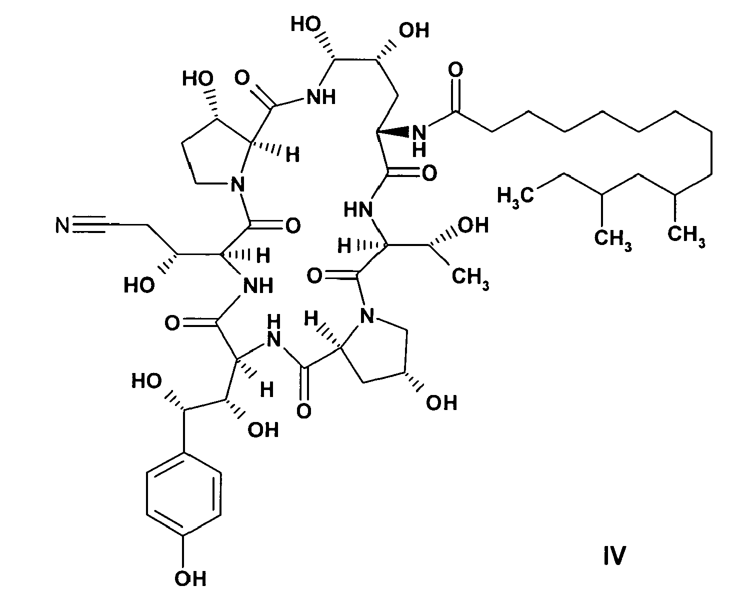
b) reacting the compound of formula IV as obtained in step a) with a thiophenol to obtain a compound of formula V 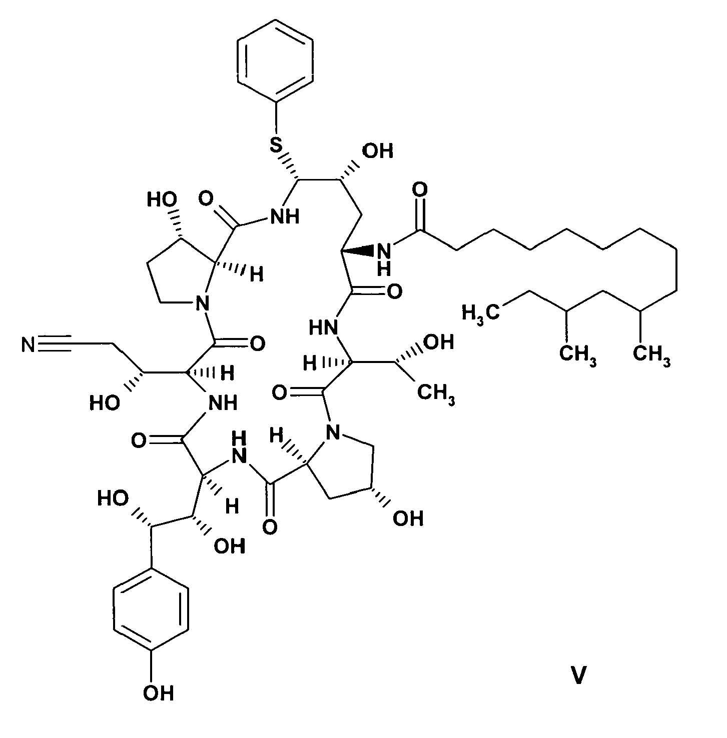
c) reacting the compound of formula V as obtained in step b) with a compound of formula HX, wherein X is as defined above,
   to obtain a compound of formula II or an acid addition salt thereof,
d) reducing a compound of formula II or an acid addition salt thereof as obtained in step c) to obtain a ccompound of formula I or a pharmaceutically acceptable salt thereof, and
e) optionally isolating the compound of formula I or a pharmaceutically acceptable salt thereof as obtained in step d).

In preferred embodiments of the above process
R₁ is H and R₂ is selected from H, C₁-C₈ alkyl, C₃-C₄ alkenyl, (CH₂)₂₋₄OH, (CH₂)₂₋₄NR₃R₄; or
R₁ is C₁-C₈ alkyl and R₂ is selected from H, C₁-C₈ alkyl,C₃-C₄ alkenyl, (CH₂)₂₋₄OH, (CH₂)₂₋₄NR₃R₄; or
R₁ is C₃-C₄ alkenyl and R₂ is selected from H, C₁-C₈ alkyl,C₃-C₄ alkenyl, (CH₂)₂₋₄OH, (CH₂)₂₋₄NR₃R₄; or
R₁ is (CH₂)₂₋₄OH and R₂ is selected from H, C₁-C₈ alkyl,C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₈ alkyl,C₃-C₄ alkenyl, (CH₂)₂₋₄OH, (CH₂)₂₋₄NR₃R₄.

In a further preferred embodiment of the above process X is HN-CH₂-CH₂-NH₂.

In a further preferred embodiment of the above process aza cyclohexapeptide compounds of formula I are produced wherein
X is NR₁R₂ or OR₁;
and wherein when X is NR₁R₂
R₁ is H, C₁-C₄ alkyl, C₃-C₄ alkenyl, (CH₂)₂₋₄OH or (CH₂)₂₋₄NR₃R₄;
R₂ is H, C₁-C₄ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₄ alkyl;
R₄ is H or C₁-C₄ alkyl;
and wherein when X is OR₁
R₂, R₃ and R₄ are as defined above with the proviso that R₁ is not H;

In preferred embodiments of the above process
R₁ is H and R₂ is selected from H, C₁-C₄ alkyl, C₃-C₄ alkenyl, (CH₂)₂₋₄OH, (CH₂)₂₋₄NR₃R₄; or
R₁ is C₁-C₄ alkyl and R₂ is selected from H, C₁-C₄ alkyl,C₃-C₄ alkenyl, (CH₂)₂₋₄OH, (CH₂)₂₋₄NR₃R₄; or
R₁ is C₃-C₄ alkenyl and R₂ is selected from H, C₁-C₄ alkyl, C₃-C₄ alkenyl, (CH₂)₂₋₄OH, (CH₂)₂₋₄NR₃R₄; or
R₁ is (CH₂)₂₋₄OH and R₂ is selected from H, C₁-C₄ alkyl,C₃-C₄ alkenyl, (CH₂)₂₋₄OH, (CH₂)₂₋₄NR₃R₄; or
R₁ is (CH₂)₂₋₄NR₃R₄ and R₂ is selected from H, C₁-C₄ alkyl, C₃-C₄ alkenyl, (CH₂)₂₋₄OH, (CH₂)₂₋₄NR₃R₄.

Step a), i.e. reacting a compound of formula III with a dehydrating agent to obtain a compound of formula IV may be carried out according to methods known in the art, e.g. under conditions known for such type of reaction, for instance as described in EP535967A. Suitable reagents to dehydrate the amide of formula III are anhydrides such as acetic anhydride, trifluoroacetic anhydride and phosphorus pentoxide, acid chlorides such as oxalylchloride, phosphorus oxychloride etc, phosphonium reagents such as triphenylphosphoniumchloride, carbodiimides such as dicyclohexylcarbodiimide or other dehydrating agents such as aluminium chloride or titanium tetrachloride.
Preferably, cyanuric chloride as described e.g. in EP535967A is used.

Step b), i.e. reacting the compound of formula IV with a thiophenol to obtain a compound of formula V may be carried out in analogy, e.g. according to methods known in the art, for example as disclosed in EP912603A. Accordingly, a compound of formula IV may be reacted with a thiophenol in acetonitrile and trifluoroacetic acid to produce a thiophenol containing intermediate of formula V.
Any moderate strength acid, e.g. trifluoroacetic acid, phosphoric acid or trichloroacetic acid is expected to yield the intermediate of formula V in good yield. Other mercaptans such as 4-methoxythiophenol, 2-mercapto-1-methylimidazole and the like may also be used.
Preferably, thiophenol is used.

Step c), i.e. substituting the thiophenol of a compound of formula V with a nucleophilic compound of formula HX may be carried out in analogy, e.g. according to methods known in the art, for example as disclosed in EP535967A. For instance, the compound of formula V may be reacted with the corresponding alkohol (HX) in the presence of an acid, e.g. a mineral acid or an organic sulfonic acid such as e.g. camphersulfonic acid. Likewise, the thiophenyl group may be displaced e.g. by an amino group by reaction with the corresponding amine, preferably by displacement with neat ethylendiamine.

Step d), i.e. reducing a compound of formula II or an acid addition salt thereof to obtain a compound of formula I or a pharmaceutically acceptable salt thereof may be carried out as described above.

Step e), i.e. isolating a compound of formula I or a pharmaceutically acceptable salt thereof may be carried out according to methods known in the art or as as described before.

Optionally, X may be protected in order to archieve the desired regioselectivity , e.g if X contains several reactive nuclephilic groups, by protective groups such as amine or hydroxyl protecting groups. Said protective groups may be removed before, simultaneously or after the reduction step.

Preferably, in step c) as described above the compound of formula HX is ethylendiamine leading to Caspofungin after reduction according to step d) as described above.

Accordingly, in another aspect the present invention relates to a compound of formula VI 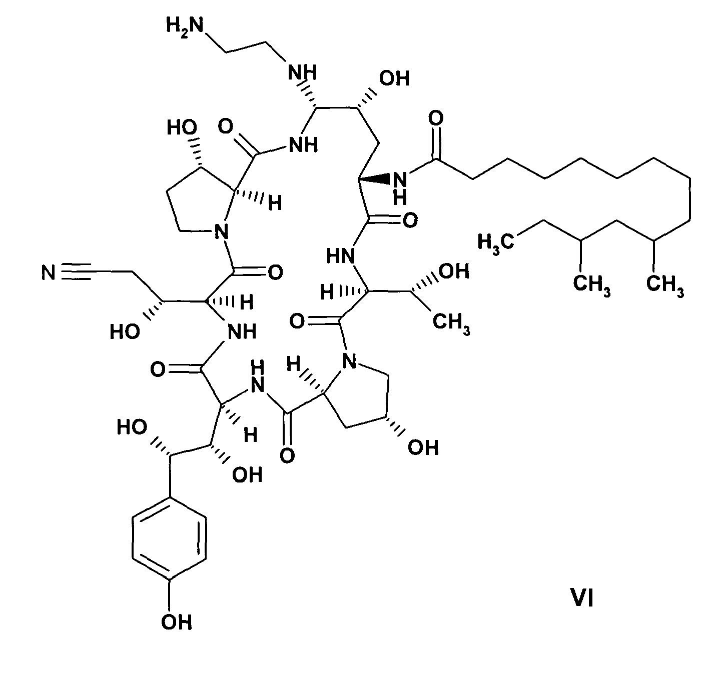 or an acid addition salt or a solvate thereof. This compound is a valuable intermediate in the preparation of Caspofungin due to its enhanced stability as it may be obtained in crystalline form as a monoaddition salt with acetic acid. Accordingly, the monoacetate of the compound of formula VI (= compound of formula VIa) is a preferred embodiment of the present invention.

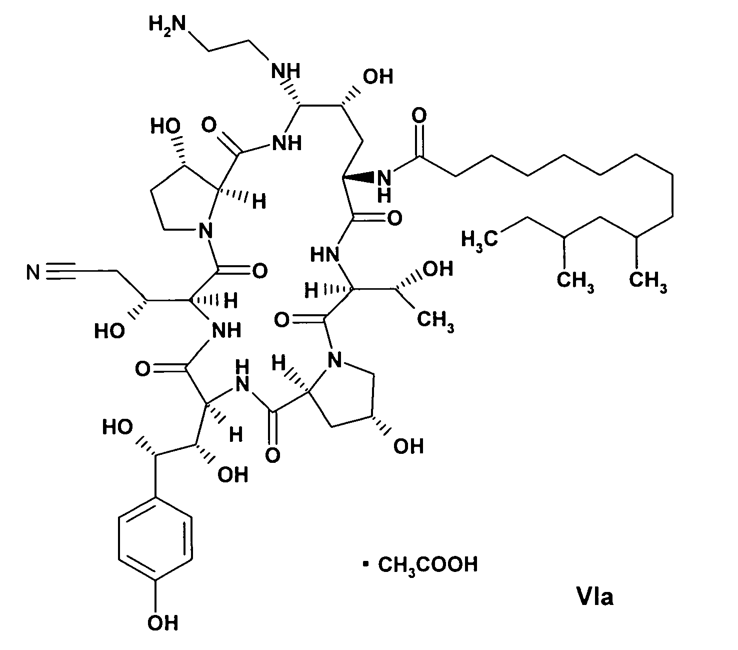

The compound of formula VI may be isolated from the reaction mixture by chromatography on e.g. RP-18 material followed by lyophilisation of rich cut fractions. The amorphous compound of formula VI may be then dissolved in an organic solvent , e.g an alcohol such as e.g. methanol or ethanol in the presence of acetic acid. The monoacetate of formula VI may be crystallized by the addition of an antisolvent such as an ester, e.g. an acetic acid C₁-C₄ alkylester such as e.g. ethylacetate. In addition, impurities such as the epimer at C₃₅ are removed efficiently by the crystallization process, impurities which are difficult or almost impossible to be removed by chromatography in an economic way.

Accordingly, in a further aspect the present invention relates to the use of a compound of formula VI or an addition salt or a solvate thereof, or its monoacetate in the preparation of caspofungin.

In a further embodiment, the present invention relates to a process for the production of a compound of formula VI or an acid addition salt or a solvate thereof comprising the steps of
a) reacting a compound of formula III as described above with a dehydrating agent to obtain a compound of formula IV as described above,
b) reacting the compound of formula IV as obtained in step a) with a thiophenol to obtain a compound of formula V as described above,
c) reacting the compound of formula V as obtained in step b) with H₂N-CH₂-CH₂-NH₂ to obtain a compound of formula VI or an acid addition salt or a solvate thereof, and
d) optionally isolating the compound of formula VI or an acid addition salt or a solvate thereof as obtained in step c).

Preferably, the compound of formula VI is isolated in step d) above its monoacetate salt, i.e. as compound of formula VIa.

The following Examples will illustrate the present invention but are not intended to limit the present invention in any way. All temperatures are given in degree Celsius and are uncorrected.

### Example 1: Preparation of the compound of formula IV

Pneumocandin Bo (10.2 g) is dissolved in a mixture of dry 1-methyl-2-pyrrolidon (90 ml) and dry N,N-dimethylformamide (10 ml). The pale yellow solution is chilled to -20 °C and cyanuric chloride (4.2 g) is added in one portion. The mixture is stirred at -20 °C until a 98 % conversion is reached (HPLC, ca. 3.5 hours). Water (100 ml) is added over 10 minutes, and the mixture is warmed to ambient temperature.

The crude mixture is slowly poured into vigorously stirred water (1400 ml). The suspension is aged for 2 hours and then filtered. The product is thoroughly washed with water and then dried under vacuum. This material (9.3 g) is used in the next step without further purification.

### Example 2: Preparation of the compound of formula IV

Pneumocandin Bo (10.0 g) is dissolved in dry N,N-dimethylformamide (100 ml). The water content of the solution is determined and is adjusted to ca. 0.15 %. The solution is chilled to - 20 °C and cyanuric chloride (4.2 g) is added in one portion. The mixture is stirred at - 20 °C until a 97 % conversion is reached (HPLC, ca. 1.0 hours). Water (100 ml) is added over 10 minutes, and the mixture is warmed to ambient temperature.
The crude mixture is slowly poured into vigorously stirred water (1400 ml). The suspension is aged for 2 hours and then filtered. The product is thoroughly washed with water and then dried under vacuum. This material (8.2 g) is used in the next step without further purification.

### Example 3: Preparation of Caspofungin

The compound of formula VI (500 mg) is dissolved in a 9:1 mixture of 2-propanol/water (13 ml) and acetic acid (650 µl). The mixture is treated with activated charcoal (50 mg) and filtered. To the filtrate is added ammonium acetate (1.35 g) and Rh/Al₂O₃ (5 % Rhodium, 105 mg). The resulting mixture is treated with hydrogen (atmospheric pressure) at room temperature for approximately 24 hours. Activated carbon (100 mg) is added and the mixture is filtered. The filtrate is evaporated under reduced pressure. The residue is dissolved in methanol (10 ml) and water (50 ml) and loaded on a preparative C-18 column. The product is eluted with 22 % acetonitril/water (0.15 % acetic acid). The rich cut fractions are pooled and lyophilized to give caspofungin diacetate (291 mg) as an amorphous white solid.

### Example 4: Preparation of Caspofungin

The compound of formula VI (250 mg) is dissolved in a 8:2 mixture of 2-propanol/water (25 ml) and acetic acid (325 µl). The mixture is treated with activated charcoal (25 mg) and filtered. To the filtrate is added Pd/C (10 % Palladium, 250 mg) and ammonium formiate (2.03 g). The resulting mixture is stirred vigorously at room temperature for approximately 24 hours. The mixture is filtered. The filtrate is diluted with water and loaded on a preparative C-18 column. The product is eluted with 22 % acetonitrile/water (0.15 % acetic acid). The rich cut fractions are pooled and lyophilized to give caspofungin diacetate (94 mg) as an amorphous white solid.

### Example 5: Preparation of Caspofungin

The compound of formula VI (20 g) is dissolved in a 85:15 mixture of 2-propanol/water (284 ml) and acetic acid (20 ml). Ammonium acetate (50 g) and Rh/Al₂O₃ (5 % Rhodium, 2 g) are added. The resulting mixture is treated with hydrogen (1 bar) at 30 °C for approximately 7.5 hours. Then the catalyst is filtered off. The filtrate is stirred with a metal scavenging agent (e.g. MSA-FC C-1, 17 g) for 2 hours at 30 °C and then filtered again. The filter cake is washed with 2-propanol (3 times 20 ml each). The filtrate and washings are combined and evaporated under reduced pressure. The residue is dissolved in methanol (250 ml) and water (1250 ml) and purified by preparative HPLC using a reversed phase C-8 column. The product is eluted with 22 % acetonitrile/water (0.15 % acetic acid). The rich cut fractions are pooled and lyophilized to give caspofungin diacetate (13.7 g) as an amorphous white solid.

The solid (2 g) is dissolved in ethanol (21.7 ml) and water (2.35 ml) at 25 °C. Undissolved material is removed by filtration. To the filtrate is added acetic acid (122 µl) and subsequently ethyl acetate (17.5 ml) is added slowly (2 hours). The solution is seeded and stirred for 1 hour at 25 °C. Another portion of ethyl acetate (22.5 ml) is added during 5 hours and the crystal suspension is aged for 1 hour. The crystalline solid is filtered off and washed with a mixture of ethanol/water/ethyl acetate (22 ml/2.5 ml/40 ml). The wet cake is dried in a stream of nitrogen to yield 1.7 g of caspofungin diacetate.

### Example 6: Preparation of the compound of formula V

6.0 g of compound of formula IV is suspended in 240 ml dry acetonitrile and cooled to -15°C. 2.6 g thiophenol are added. 12.7 ml trifluoroacetic acid are added at a temperature below - 10°C. The reaction mixture is stirred at -15 to -10°C until the content of compound of formula IV is lower than approximately 3% (HPLC) in the reaction mixture. 581 ml cold water are added to the reaction suspension in 1h. The precipitate is isolated and washed with acetonitrile/water 3:1 till the washing solution has a pH above 5.
5.6 g of compound of formula V are obtained. This material is used in the next step without further purification.

### Example 7: Preparation of the compound of formula VI

15.0 g of compound of formula V are added to 42.6 ml neat ethylenediamine and stirred at ambient temperature until the starting material is completely consumed. 72 ml methanol are added at a temperature of below 25°C. Subsequently 204 ml acetic acid (65%) are added at the same temperature. Further 206 ml water are added.
The yellowish solution is extracted with 140 ml n-heptane. The layers are separated and the organic layer is back-extracted using 38 ml acetic acid (65%). The aqueous layers are filtrated and chromatographed using 0.15% acetic acid/ acetonitrile 70/30. The rich cuts are combined and lyophilized. 7.65 g of the compound of formula VI are obtained as acetate adduct.

### Example 8: Crystallization of the compound of formula VI as mono acetate addition salt

69.9 g of amorphous compound of formula VI with an assay of 80% (HPLC) and a content of the epimer at C35 of 3.0% (HPLC) prepared as described in example 5 are dissolved in 760 ml of methanol and 4.3 ml of glacial acetic acid at ambient temperature. 645 ml of ethyl acetate are added during 1 h. The mixture is seeded and stirred for another hour. After addition of another 1.2 1 of ethyl acetate during 2h the crystalline product is isolated by filtration. The filter cake is washed with a mixture of 183 ml of methanol, 490 ml of ethyl acetate and 16 ml of water. The product is dried in vacuo yielding 53 g of crystalline compound of formula VI as monoacetate addition salt (yield 88%).

Assay 93.1 % (HPLC)
Water content: 3.0 %
Impurity C35-epimer: 0.1%
MS (LC-MS, ESI)
1098.7 [M+H]
¹H-NMR (CD₃OD, 300 MHz)
7.15 (d, J=8.5Hz, 2H), 6.78 (d, J=8.5Hz, 2H), 4.55 (m, 5H), 4.3 (m, 5H), 4.19 (d, J=4.5Hz, 1H), 4.1-3.75 (m, 5H)), 3.15-2.65 (m, 6H), 2.45 (m, 1H), 2.35-1.85 (m, 8H), 1.93 (s, 3H), 1.7-0.8 (m, 36H)
¹³C NMR (CD₃OD, 75 MHz)
10.59, 18.5, 19.2, 19.74, 22.39, 22.86, 26.1, 27.01, 29.32, 29.35, 29.54, 29.72, 30.11, 30.21, 31.88, 33.62, 35.98, 37.04, 37.5, 39.04, 42.24, 44.9, 46.02, 49.96, 53.89, 54.92, 56.13, 57.6, 61.65, 63.06, 67.27, 68.31, 68.77, 69.48, 70.33, 74.04, 74.67, 76.21, 115.27, 118.71, 128.71, 131.96, 157.46, 167.28, 171.76, 171.88, 172.5, 172.66, 172.85, 175.22, 178.82

## Claims

1. A compound of formula VI 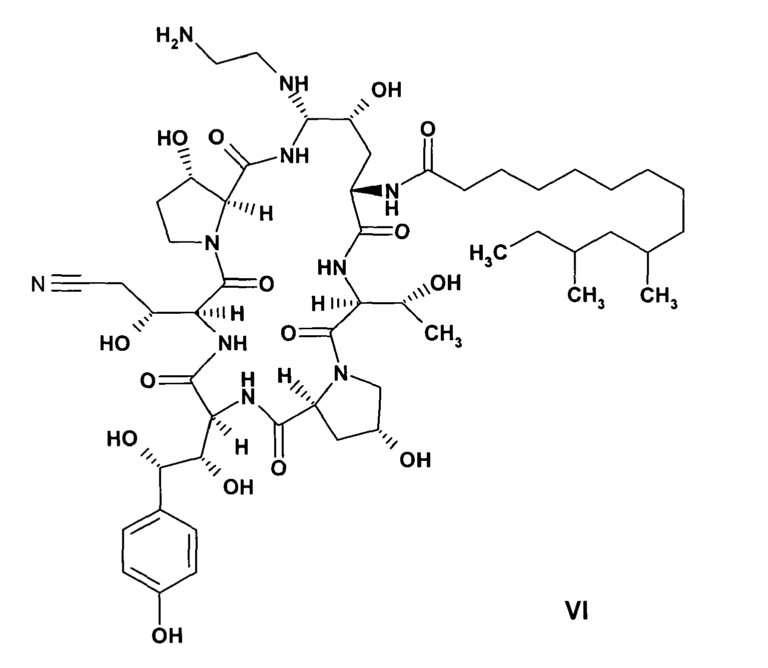 or an addition salt or a solvate thereof.

2. A compound of formula VIa

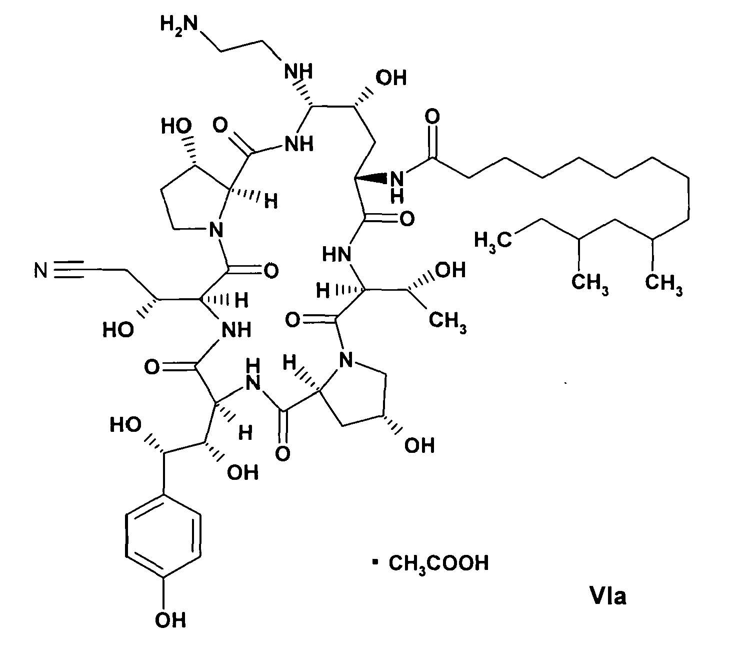

3. A process for the production of aza cyclohexapeptide compounds of formula I 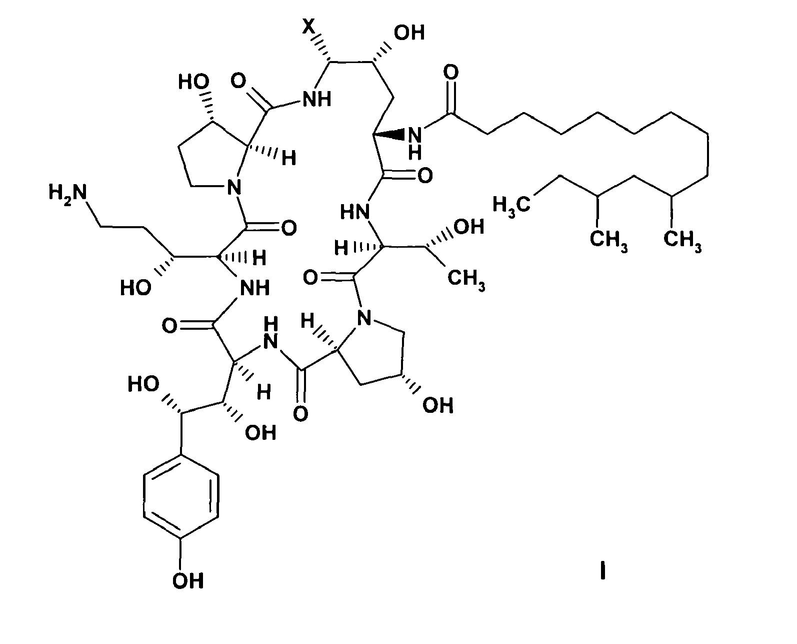 wherein
X is NR₁R₂ or OR₁;
and wherein when X is NR₁R₂
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄; or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
and wherein when X is OR₁
R₂, R₃ and R₄ are as defined above with the proviso that R₁ is not H;
or a pharmaceutically acceptabl salt thereof comprising the steps of
a) reducing a compound of formula II or an acid addition salt thereof 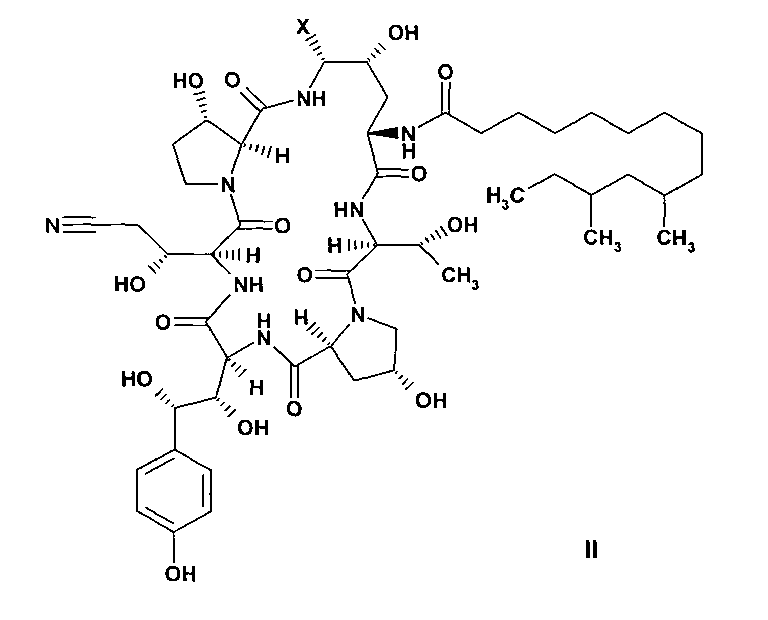 wherein X is defined as above,
to obtain a compound of formula I or a pharmaceutically acceptable salt thereof, and
b) optionally isolating the compound of formula I or a pharmaceutically acceptable salt thereof as obtained in step a).

4. A process for the production of aza cyclohexapeptide compounds of formula I 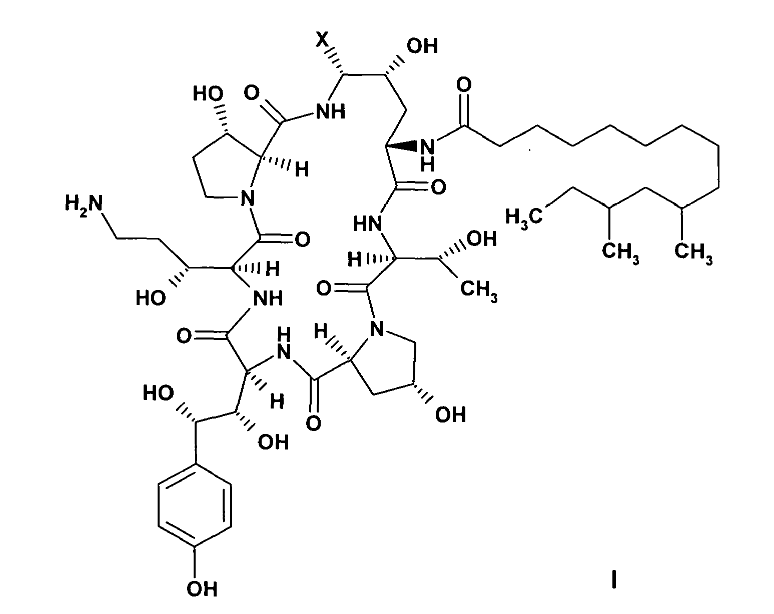 wherein
X is NR₁R₂ or OR₁;
and wherein when X is NR₁R₂
R₁ is H, C₁-C₈ alkyl, C₃-C₄ alkenyl, (CH₂)₂₋₄OH or (CH₂)₂₋₄NR₃R₄;
R₂ is H, C₁-C₈ alkyl, C₃-C₄ alkenyl, (CH₂)₂₋₄OH, (CH₂)₂₋₄NR₃R₄, or
wherein NR₁R₂ form a heterocyclic ring and R₁ and R₂ together are (CH₂)₄, (CH₂)₅, (CH₂)₂O(CH₂)₂ or (CH₂)₂NH(CH₂)₂;
R₃ is H or C₁-C₈ alkyl;
R₄ is H or C₁-C₈ alkyl;
and wherein when X is OR₁
R₂, R₃ and R₄ are as defined above with the proviso that R₁ is not H;
or a pharmaceutically acceptable salt thereof comprising the steps of
a) reacting a compound of formula III 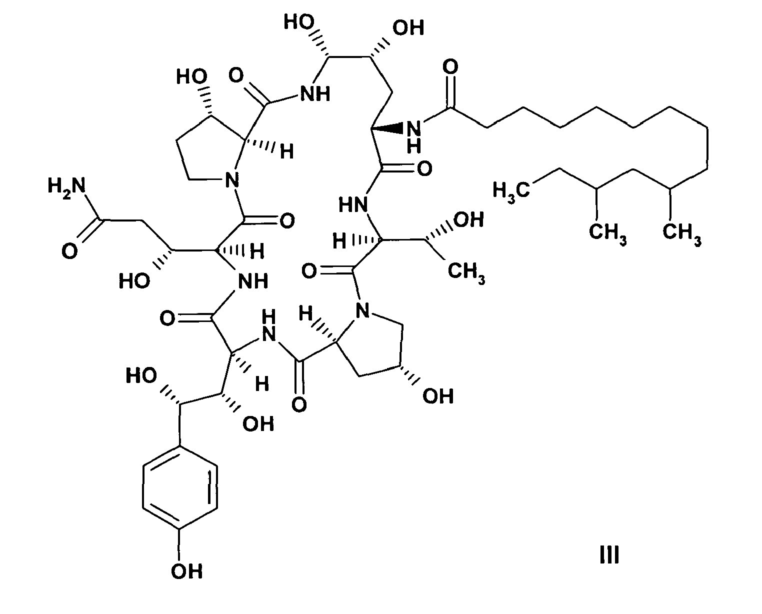 with a dehydrating agent to obtain a compound of formula IV 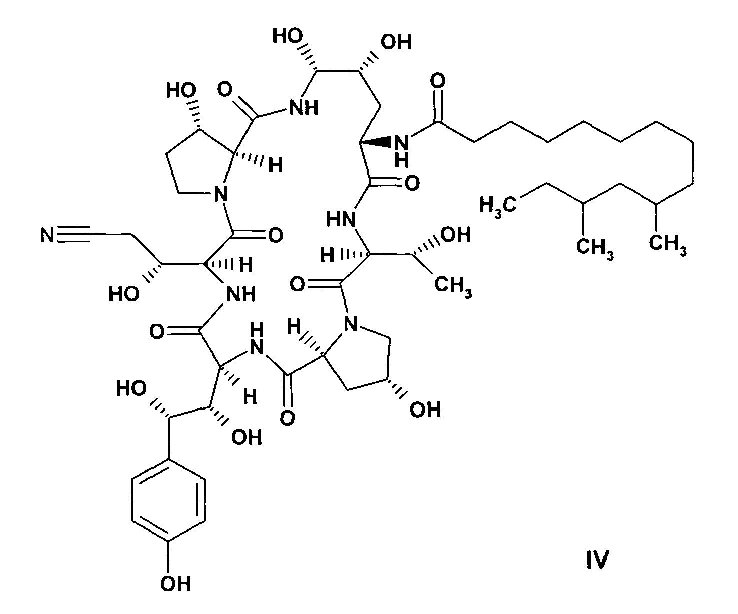
b) reacting the compound of formula IV as obtained in step a) with a thiophenol to obtain a compound of formula V 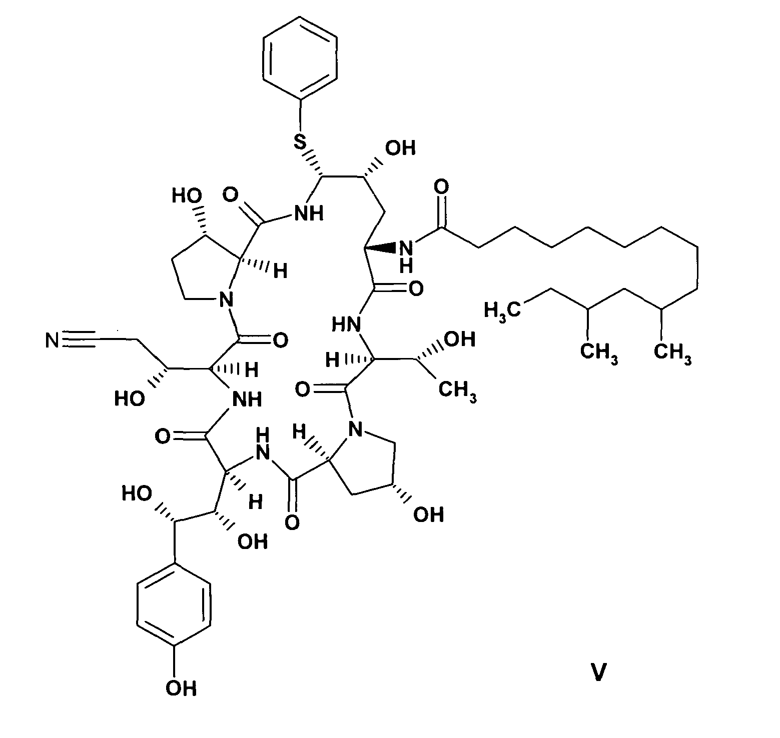
c) reacting the compound of formula V as obtained in step b) with a compound of formula HX, wherein X is as defined above,
to obtain a compound of formula II or an acid addition salt thereof,
d) reducing a compound of formula II or an acid addition salt thereof as obtained in step c) to obtain a compound of formula I or a pharmaceutically acceptable salt thereof, and
e) optionally isolating the compound of formula I or a pharmaceutically acceptable salt thereof as obtained in step d).

5. A process for the production of a compound of formula VI or an acid addition salt or a solvate thereof comprising the steps of
a) reacting a compound of formula III 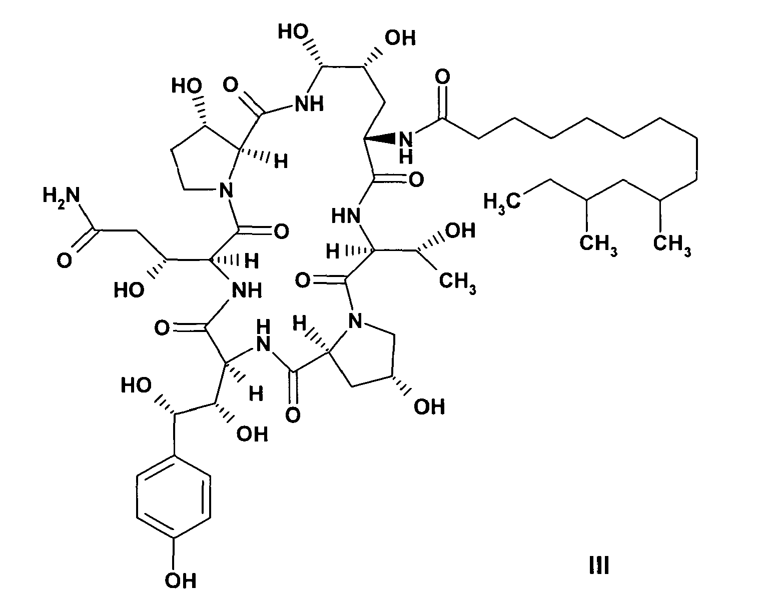 with a dehydrating agent to obtain a compound of formula IV 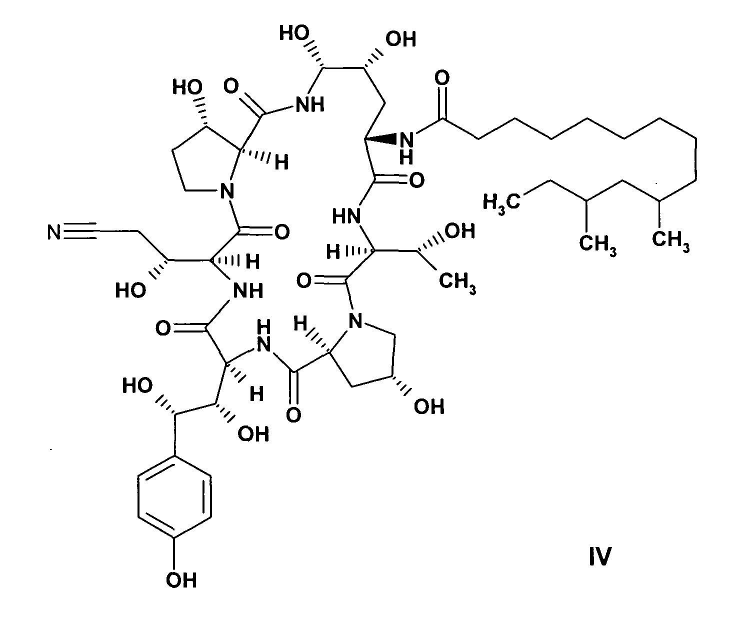
b) reacting the compound of formula IV as obtained in step a) with a thiophenol to obtain a compound of formula V 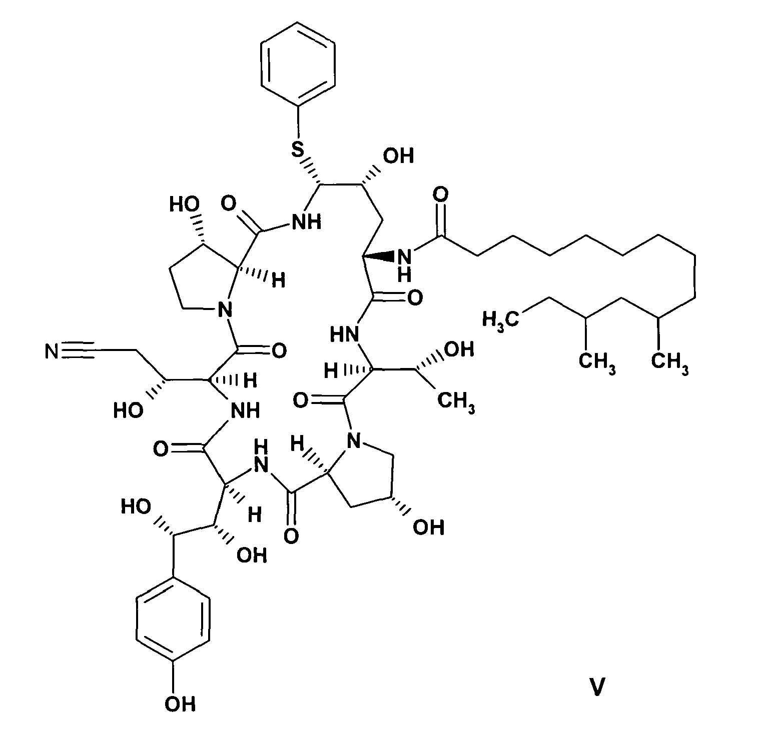
c) reacting the compound of formula V as obtained in step b) with H₂N-CH₂-CH₂-NH₂ to obtain a compound of formula VI or an acid addition salt or a solvate thereof, and
d) optionally isolating the compound of formula VI or an acid addition salt or a solvate thereof as obtained in step c).

6. A process according to claim 5 wherein the compound of formula VI in step d) is isolated in its monoacetate salt form.

7. A process according to claim 3 or 4 wherein X is HN-CH₂-CH₂-NH₂.

8. A process according to claim 3 or 4 wherein the reduction step is carried out by catalytic hydrogenation.

9. A process according to claim 8 wherein Rh/Al₂O₃ or Pd on carbon is used as catalyst.

10. A process according to any one of claims 3 or 4 and 7 to 9 wherein in the reduction step H₂ is used as hydrogen source.

11. A process according to claim 4 or 5 wherein cyanuric chloride is used as dehydrating agent in step a).

12. Use of a compound according to claim 1 or 2 in the preparation of caspofungin.
